(19) Europäisches Patentamt
European Patent Office
Office européen des brevets

(11) **EP 1 562 047 B1**

(12) **EUROPEAN PATENT SPECIFICATION**

(45) Date of publication and mention
of the grant of the patent:
**14.05.2008 Bulletin 2008/20**

(51) Int Cl.:
*G01N 33/86* (2006.01)     *G01N 33/92* (2006.01)

(21) Application number: **05002403.3**

(22) Date of filing: **04.02.2005**

(54) **Reagent kit for detecting lupus anticoagulant**

Reagenziensatz zum Nachweis von Lupus-Antikoagulant.

Trousse de réactifs pour détecter d'anticoagulant de lupus.

(84) Designated Contracting States:
**AT BE BG CH CY CZ DE DK EE ES FI FR GB GR HU IE IS IT LI LT LU MC NL PL PT RO SE SI SK TR**

(30) Priority: **06.02.2004 JP 2004031372**

(43) Date of publication of application:
**10.08.2005 Bulletin 2005/32**

(73) Proprietor: **SYSMEX CORPORATION**
**Kobe-shi,**
**Hyogo 651-0073 (JP)**

(72) Inventor: **Okuda, Masahiro**
**Kobe-shi,**
**Hyogo 651-2214 (JP)**

(74) Representative: **HOFFMANN EITLE**
**Patent- und Rechtsanwälte**
**Arabellastrasse 4**
**81925 München (DE)**

(56) References cited:
**EP-A- 1 385 003**

- **JACOBSEN E M ET AL: "The evaluation of clotting times in the laboratory detection of lupus anticoagulants" THROMBOSIS RESEARCH, TARRYTOWN, NY, US, vol. 104, no. 4, 15 November 2001 (2001-11-15), pages 275-282, XP002261843 ISSN: 0049-3848**
- **KELSEY P R ET AL: "THE DIAGNOSIS OF LUPUS ANTICOAGULANTS BY THE ACTIVATED PARTIAL THROMBOPLASTIN TIME - THE CENTRAL ROLE OF PHOSPHATIDYL SERINE" THROMBOSIS AND HAEMOSTASIS, STUTTGART, DE, vol. 52, no. 2, 1984, pages 172-175, XP008024549 ISSN: 0340-6245**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

FIELD OF THE INVENTION

The present invention relates to a blood coagulation reagent kit which is used for performing the diagnosis of antiphospholipid syndrome (APS) in the fields of clinical chemistry, pharmaceutical studies, and more particularly, the present invention relates to a reagent kit which is capable of specifically detecting the lupus anticoagulant positive diseases by a blood test *in vitro.*

BACKGROUND

Lupus anticoagulant (hereinafter, abbreviated as LA) is an anticoagulant factor which has been first reported involving with the SLE (Systemic Lupus Erythematosus) patient. While the frequency that LA is detected in the SLE patients is a frequency of 5% through 10%, on the other hand, there is a possibility that the above-described LA is sometimes detected in the other autoimmune diseases and neoplastic diseases. Since LA is detected at a high frequency in the antiphospholipid syndrome; APS such as thrombosis, abortion and premature labor or thrombocytopenia, and the time in which LA is coagulated in the phospholipid-dependent coagulation reaction is extended, it is considered that LA is an autoantibody with respect to phospholipid.

Moreover, recently it has been clarified that LA is a heterogeneous antibody and is an antibody to a complex of negatively charged phospholipid and beta 2-glycoprotein I (beta2-GPI) or prothrombin. It is considered that the anticoagulation action of LA occurs by the process that the change in which prothrombin is changed into thrombin is inhibited by LA binding to this complex.

As a reagent for detecting LA having such characteristics, conventionally, a coagulant reagent containing a certain amount of phospholipid, for example, cephalin derived from rabbit brain, cephalin derived from bovine brain, soybean lecithin or snake venom have been used. Since if LA exists in the specimen blood, LA neutralizes phospholipid contained in the reagent, therefore, phospholipid which is necessary to the start of the cascade reaction in which the specimen is coagulated becomes inadequate, the coagulation time of the respective tests are extended in proportion to the amount of phospholipid neutralized by LA, and by which LA is capable of being diagnosed as LA positive.

Moreover, since less the amount of phospholipid in the coagulation reagent, the more the detection sensibility of LA is enhanced, it has been performed that the reagent is diluted and/or more than two different methods are combined in order to contemplating the enhancement of the accuracy of the test. For example, the method in which activated partial thromboplastin time measurement (hereinafter, abbreviated as APTT) in which two kinds of reagents that are different in the respect of concentration of phospholipid are combined is used and Rosner Index and Lupus Ratio (LR value) are calculated from the coagulation time of the respective reagents and LA positive ratio is determined is known.

As a coagulation measurement reagent which is available in the market, there is a test reagent which is called as Gradipore LA (made by Gradipore Co., Ltd.: Australia) that performs the test by utilizing Russell's viper venom method. It is a reagent kit in which the first reagent containing snake venom and the second reagent containing snake venom and an excessive amount of soybean phospholipid are combined, and in the case where the ratio that is found by the formula of the coagulation time at the time when the first reagent was added / the coagulation time at the time when the second reagent was added is 1.3 or more, it is defined that it is determined as LA positive.

Moreover, according to the research reports which have been already reported, the detection of LA has been performed by arithmetical calculation by utilizing the difference between the concentrations of phospholipid using phospholipid derived from rabbit brain and the like (V. Chantarangkul, et al., Thromb. Res. 1992, 67: 355-365; E. Rosner, et al., Thromb. Haemost. 1987, 57: 144-147).

And furthermore, the method in which the coagulation time at the time when APTT reagent which has been combined with two kinds of reagents whose phospholipid concentrations are different, or diluted Russell's viper venom measurement reagent was used is measured using the specimen sample in which normal plasma and plasma of the patient have been mixed at the ratio of 1:1 and LA is detected by means of making the degree that the coagulation time of the plasma of the patient is adjusted by the normal plasma be index has been also utilized.

In EP-A-1385003 reagent kits are disclosed for detecting lupus coagulant comprising a first coagulation time reagent containing PS; and a second coagulation time measurement reagent containing PS, wherein the content of PS to the total content of phospholipids in the first coagulation time reagent is different from the content of PS to the total content of phospholipids in the first coagulation time reagent (10 to 20 times difference). According to the description additionally other phospholipids such as PE can be present in the first and second coagulation time reagent, however there is no direct and unambiguous disclosure of different concentrations of PE being present in the first and second coagulation time measurement reagents.

In the phospholipid-dependent coagulation measurement which has been conventionally performed, in any case of it, phospholipid derived from the natural resource has been used, and the component of the phospholipid has not been adjusted. There is a problem that since the phospholipid derived from the natural source is a heterogenous one, the property has influence upon the coagulation time, and the detection sensitivities of LA is considerably various. Moreover, there is also a problem that although in the relevant coagulation test, it is possible to normalize the coagulation time by replenishing the deleted coagulation factors by mixing the specimen without LA with the normal plasma, it is difficult to determine the specimen containing the blood coagulation inhibitor such as warfarin or heparin from the plasma of the LA positive patient and also in the case of the specimens containing these coagulation inhibitors, there is a possibility that these are determined as LA positive.

Therefore, in order to accurately determine LA diseases, it is necessary to make a distinction between the extension of the coagulation time because of the coagulation activation inhibitor except for LA and coagulation factor deficiency, and the extension of the coagulation time because of LA.

SUMMARY

An object of the present invention is to provide a reagent kit in which the detection sensitivity of LA is enhanced and the specimen is capable of being specifically detected even if it is a LA weak positive specimen as well as the reagent makes a distinction between the plasma of the patient to whom heparin has been given and the plasma of the patient to whom warfarin has been given.

The first aspect of the present invention relates to a reagent kit for detecting lupus anticoagulant comprising a first coagulation time measurement reagent containing phospholipid including phosphatidylserine and phosphatidylethanolamine and a second coagulation time measurement reagent containing phospholipid including phosphatidylserine and phosphatidylethanolamine, wherein the concentration of phosphatidylethanolamine of said first coagulation time measurement reagent is in the range from 40 to 280 $\mu$M, and the concentration of phosphatidylethanolamine contained in said second coagulation time measurement reagent is in the range from 1 to 30 $\mu$M.

The second aspect of the present invention relates to a reagent kit for detecting lupus anticoagulant comprising a first coagulation time measurement reagent comprising a first partial reagent and a second partial reagent and a second coagulation time measurement reagent comprising a third partial reagent and a fourth partial reagent, wherein said first partial reagent contains phospholipid including phosphatidylserine and phosphatidylethanolamine said second partial reagent contains a calcium ion and said third partial reag contains phospholipid including phosphatidylserine and phosphatidylethanolamine and said fourth partial reagent contains a calcium ion and the concentration of including phosphatidylserine and phosphatidylethanolamine and the concentration of phosphatidylethanolamine contained in said first partial reagent is in the range from 40 to 280 $\mu$M, and the concentration of phosphatidylethanolamine contained in said third partial reagent is in the range from 1 to 30 $\mu$M.

BRIEF DESCRIPTION OF DRAWING

Fig.1 is a graph showing the relationship between PE concentration and LR value contained in the first partial reagent; and

Fig.2 is a graph showing the relationship between PS concentration and LR value contained in the first partial reagent.

DESCRIPTION OF THE PREFERRED EMBODIMENT

As a result of diligently considering these in order to solve the above-described problems, the present inventors contemplate to provide a reagent kit for detecting LA in which the detection sensitivity of LA is enhanced and LA is capable of being specifically detected even if it is LA weak positive specimen as well as it makes a distinction between LA specimen and the plasma of the patient to whom heparin has been given, the plasma of the patient to whom warfarin has been given by adjusting the containing amount of phosphatidylethanolamine in the reagents whose containing amounts of phosphatidylserine with respect to phosphalipid contained the reagents are different.

The reagent kit for detecting lupus anticoagulant comprising a first coagulation time measurement reagent containing phospholipid including phosphatidylserine and phosphatidylethanolamine and a second coagulation time measurement reagent containing phospholipid including phosphatidylserine and phosphatidylethanolamine, wherein the concentration of phosphatidylethanolamine of said first coagulation time measurement reagent is in the range from 40 to 280 $\mu$M, and the concentration of phosphatidylethanolamine contained in said second coagulation time measurement reagent is in the range from 1 to 30 $\mu$M.

The first coagulation time measurement reagent containing phospholipids including phosphatidylserine and phosphatidylethanolamine and the second coagulation time measurement reagent containing phospholipids including phosphatidylserine and phosphatidylethanolamine, wherein the content of the phosphatidylserine to the total content of the phospholipids in the first coagulation time measurement reagent is different from the content of the phosphatidylserine to the total content of the phospholipids in the second coagulation time measurement reagent.

Although phosphatidylethanolamine (hereinafter, may be abbreviated as PE) and phosphatidylserine (hereinafter, may be abbreviated as PS) contained in the above-described first coagulation time measurement reagent and second coagulation time measurement reagent may be naturally derived ones, there is a problem that since the phospholipids derived from the natural resource is heterogeneous, by which the coagulation time is influenced, and the detection sensitivities of LA become considerably various, therefore, it is preferable to use a synthetic one. Moreover, in the case where PE and PS derived from the natural resource are used, it is preferable that PE and PS refined to the purity of 99% or more is used from the above-described viewpoint.

Referring to phospholipids except for PE and PS contained in the above-described first coagulation time measurement reagent and second coagulation time measurement reagent, as a phospholipid for promoting the blood coagulation, phosphatidylcholine (hereinafter, may be abbreviated as PC) are listed. Although PC may be also PC derived from the natural resource, it is preferable that it is made from a synthetic one. Also in the case where PC derived from the natural resource is used, it is preferable that the PC purified to the purity of 99% or more is used.

Although a variety of the side chain of aliphatic acid of phospholipids such as PE, PS or PC are used, palmitic acid, oleic acid or stearic acid are preferably listed, and furthermore, oleic acid is more preferably listed.

Furthermore, the other components necessary for generating the blood coagulation *in vitro* may be contained in the first coagulation time measurement reagent and the second coagulation time measurement reagent. As the other components, for example, activator, snake venom, calcium, tissue factors and the like are listed. As an activator, it is preferable that one kind or more selected from the group consisting of ellagic acid, kaolin, celite and colloidal silica is used. As a tissue factor, it is preferable that a tissue factor derived from rabbit brain and human placenta, and recombinant are used.

As for these other components, these are appropriately selected according to the kinds of coagulation time measurement reagents. For example, in the case where the first coagulation time measurement reagent and the second coagulation time measurement reagent contain calcium and activator, the coagulation time is measured based on the measurement principle of activated partial thromboplastin time measurement. In the case where the first coagulation time measurement reagent and the second coagulation time measurement reagent contain calcium and snake venom, the coagulation time is measured based on the principle of Russell's viper venom measurement. Moreover, in the case where the first coagulation time measurement reagent and the second coagulation time measurement reagent contain calcium and a tissue factor, the coagulation time is measured based on the principle of prothrombin time measurement.

It should be noted that buffers such as HEPES or TRIS might be contained if it is necessary. As for the concentration of a buffer, the concentration generally used in the clinical chemistry may be available, it can be determined by performing a simple repeated experiment.

As for the contents of phospholipid contained in the first coagulation time measurement reagent and the second coagulation time measurement reagent, in the measurement method in which the coagulation time is measured by mixing the specimen and the first coagulation time measurement reagent or the second coagulation time measurement reagent in equal proportions (in the case where 50 $\mu$L of the specimen and 50 $\mu$L of me first coagulation time measurement reagent or the second coagulation time measurement reagent are mixed), these are listed as follows: the concentration of PE contained in the first coagulation time measurement reagent is in the range from 40 to 280 $\mu$M, it is preferable that it is in the range from 65 to 269 $\mu$M, and it is more preferable that it is in the range from 100 to 200 $\mu$M, the concentration of PS is in the range from 30 to 370 $\mu$M, it is preferable that it is in the range from 37 to 250 $\mu$M, and it is more preferable that it is in the range from 60 to 250 $\mu$M, and the concentration of PC is in the range from 10 to 140 $\mu$M, and it is more preferable that it is in the range from 25 to 64 $\mu$M.

Moreover, the concentration of PE contained in the second coagulation time measurement reagent is in the range from 1 to 30 $\mu$M, and it is preferable that it is in the range from 1 to 27 $\mu$M, and it is more preferable that it is in the range from 6 to 21 $\mu$M, the concentration of PS is in the range from 1 to 25 $\mu$M, and it is more preferable that it is in the range from 6 to 19 $\mu$M, and the concentration of PC is in the range from 10 to 140 $\mu$M, and it is more preferable that it is in the range from 25 to 64 $\mu$M. If the concentration of PE is low, the coagulation time is extended, therefore, it is not preferable.

Although as for the concentration of the above-described phospholipid, the concentration in the case where 50 $\mu$l of the specimen and 50 $\mu$L of the first coagulation time measurement reagent or the second coagulation time measurement reagent are mixed, in the case where the mixture ratio between the specimen and the coagulation time measurement reagents is different, it may be made so that the final concentration of phospholipid after the specimen and the first coagulation time measurement reagent or the second coagulation time measurement reagent were mixed is made similar to the above-described case.

Moreover, it is preferable that the concentration of PE or the concentration of PS contained in the first coagulation time measurement reagent is 3 to 15 times as high as the concentration of PE or the concentration of PS contained in the second coagulation time measurement reagent. If the multiplying factor is low, it is not preferable since it becomes difficult to make a distinction between the specimen of LA positive patient and the plasma of the patient to whom heparin has been given and of the patient to whom warfarin has been given. Moreover, if the multiplying factor is high, it is not preferable since the difference of the coagulation time of the normal plasma becomes large between the first coagulation time measurement reagent and the second coagulation time measurement reagent.

Moreover, the first coagulation time measurement reagent and the second coagulation time measurement reagent may be mixture of the phospholipid and the above-described other components, respectively.

Moreover, the first coagulation time measurement reagent comprises first partial reagent and a second partial reagent contained in separate containers from each other, whereas the second coagulation time measurement reagent comprises a third partial reagent and a fourth partial reagent contained in separate containers from each other. The PE concentration contained in the first partial reagent is in the range from 40 to 280 $\mu$M, it is preferable that it is in the range from 65 to 269 $\mu$M, and it is more preferable that it is in the range from 100 to 200 $\mu$M, the concentration of PS is in the range from 30 to 370 $\mu$M, it is preferable that it is in the range from 37 to 250 $\mu$M, and it is more preferable that it is in the range from 60 to 250 $\mu$M, and the concentration of PC is in the range from 10 to 140 $\mu$M, and it is more preferable that it is in the range from 25 to 64 $\mu$M.

Moreover, the concentration of PE contained in the third partial reagent is in the range from 1 to 30 $\mu$M, and it is preferable that it is in the range from 1 to 27 $\mu$M, and it is more preferable that it is in the range from 6 to 21 $\mu$M, the concentration of PS is in the range from 1 to 25 $\mu$M, and it is more preferable that it is in the range from 6 to 19 $\mu$M, and the concentration of PC is in the range from 10 to 140 $\mu$M, and it is more preferable that it is in the range from 25 to 64 $\mu$M

As for the coagulation time measurement reagent based on the principle of the activated partial thromboplastin time measurement, the reagents in which the first partial reagent and the third partial reagent contain phosphatidylserine, phosphatidylethanolamine, phosphatidylcholine and the activator, and the second partial reagent and the fourth partial reagent contain calcium as well as the first coagulation time measurement reagent comprises the first partial reagent and the second partial reagent, the second coagulation time measurement reagent comprises the third partial reagent and the fourth partial reagent are listed.

As for the coagulation time measurement reagent based on the principle of Russell's viper venom measurement, the reagents in which the first partial reagent and the third partial reagent contain phosphatidylserine, phosphatidylethanolamine, phosphatidylcholine and snake venom and the second partial reagent and the fourth partial reagent contain calcium as well as the first coagulation time measurement reagent comprises the first partial reagent and the second partial reagent, and the second coagulation time measurement reagent comprises the third partial reagent and the fourth partial reagent are listed.

As for the coagulation time measurement reagent based on the principle of prothrombin time measurement, the reagents in which the first partial reagent and the third partial reagent contain phosphatidylserine, phosphatidylethanolamine, phosphatidylcholine and tissue factor and the second partial reagent and the fourth partial reagent contain calcium as well as the first coagulation time measurement reagent comprises the first partial reagent and the second coagulation time measurement reagent comprises the third partial reagent and the fourth partial reagent are listed.

A reagent kit of the present invention is the combination of two kinds of reagents whose coagulation times are different in the first coagulation time measurement reagent comprising the first partial measurement reagent and the second partial measurement reagent and in the second coagulation time measurement reagent comprising the third partial measurement reagent and the fourth partial measurement reagent, which have the above-described components.

Next, a method in which LA is detected will be explained below using a reagent kit of the present invention having the above-described constitution.

First, the specimen sample is divided into two, the first coagulation time measurement reagent is added to one specimen sample, and the second coagulation time measurement reagent is added to the other specimen sample. In the case where the coagulation time measurement reagent has been divided into the first partial reagent and the second partial reagent, respectively, after the first partial reagent and the second partial reagent are mixed, the specimen may be added, or after at first, the first partial reagent (or the second partial reagent) is added, the second partial reagent (or the first partial reagent) may be added. In the case where the first partial reagent or the second partial reagent is separately added, the mixture of the specimen and the partial reagents may be heated if it is necessary after the first partial reagent (or the second partial reagent) is added, before the second partial reagent (or the first partial reagent) is added.

The coagulation time of the sample is measured after the respective reagents have been added to the specimen. As for the measurement of the coagulation time, it may be performed by a known method, for example, it may be measured by utilizing a known automatic measurement apparatus.

Herein, as a sample used for the specimen, it is preferable that the plasma is used rather than the blood being used as it is, and it is more preferable that the platelet removal plasma is used. And it is more preferable that the mixture in which the normal plasma or the blood platelet removal plasma has been mixed to the plasma of the patient is made a sample. Because by previously having mixed the normal plasma, the extension of the coagulation time based on the coagulation factor deficiency can be prevented, and the sensibility of the phospholipid-dependent blood coagulation test is enhanced. The mixture ratio of the plasma and the normal plasma is generally 4:1 through 1:4, and it is preferable that it is 1:1.

In the coagulation test using a reagent kit of the present invention, the coagulation time of the sample derived from the anticoagulation diseases such as LA positive patient, blood coagulation factor deficiency patient, patient to whom warfarin has been given, patient to whom heparin has been given and the like is longer than the coagulation time of the normal plasma. Furthermore, as for the specimen of LA positive patient, the difference between coagulation times using the first coagulation time measurement reagent and the second coagulation time measurement reagent is also admitted. Specifically, in the case of a sample derived from LA positive patient, the coagulation time at the time when the second coagulation time measurement reagent becomes longer than the coagulation time at the time when the first coagulation measurement reagent. On the other hand, in the case of the samples derived from the blood coagulation factor deficiency patient, the patient to whom warfarin has been given, the patient to whom heparin has been given, the significant difference of the coagulation times was not admitted between the first coagulation time measurement reagent and the second coagulation time measurement reagent. Therefore, LA can be specifically detected based on the difference of the coagulation time between the first coagulation time measurement reagent and the second coagulation time measurement reagent.

Although the detection of LA can be determined by the difference of the coagulation times which have been measured by the first coagulation time measurement reagent and the second coagulation time measurement reagent, it is preferable for the sake of determining at a higher accuracy that the determination is performed by utilizing the ratio of the coagulation time of the sample derived from the patients with respect to the coagulation time of the normal plasma, for example, Rosner Index (E. Rosner, et al., Thromb. Haemast. 1987, 57: 144-147) or Lupus Ratio (LR value) (R. Schjetlein, et al., Thromb. Res. 1993, 69:239-250).

In the case of the normal plasma, LR value which is calculated using the first coagulation time measurement reagent and the second coagulation time measurement reagent is on the order of 1. Moreover, even in the case where it is derived from the blood coagulation factor deficiency patient, the patient to whom warfarin has been given, or the patient to whom heparin has been given, the coagulation time is extended in comparison with the normal plasma, since the difference of the coagulation time between the first coagulation time reagent and the second coagulation time reagent is scarcely admitted, the LR value becomes on the order of 1. In contrast to this, in the case where LA is positive, although depending upon the respective concentrations of the first coagulation time measurement reagent and the second coagulation time measurement reagent and its combination, since the coagulation time is more extended by the first coagulation time measurement reagent than by the second coagulation time measurement reagent, the specimen derived from LA positive patient can be automatically detected by comparing the LR values.

[Examples]

(Example 1)

Consideration of concentration of PE

The first partial reagents 1-6 and the third partial reagent were prepared by mixing HEPES, ellagic acid, Tris, phophatidylserine (PS), phosphatidylethanolamine (PE) and phosphatidylcholine (PC).

Referring to the concentration of the respective components of the first partial reagent, the concentration of HEPES was 50 mM, the concentration of ellagic acid was 0.1 mM, the concentration of Tris was 25 mM, the concentration of PS was 123 $\mu$M, the concentration of PC was 38 $\mu$M, and the concentration of PE is 13 $\mu$M. Moreover, the first partial reagents 2-6 were prepared by performing similarly to the first partial reagent 1 except that the concentration of PE was changed into 67 $\mu$M, 101 $\mu$M, 134 $\mu$M, 202 $\mu$M, and 269 $\mu$M. The pH of the first partial reagents 1-6 was 7.35.

Referring to the concentration of the respective components of the third partial reagent, the concentration of HEPES was 50 mM, the concentration of ellagic acid was 0.1 mM, the concentration of Tris was 25 mM, the concentration of PS was 12 $\mu$M, the concentration of PE was 13 $\mu$M, and the concentration of PC was 38 M. It should be noted that a synthetic phospholipid whose aliphatic acid side chain is oleic acid was used for PE, PS and PC.

Moreover, the reagents prepared by purified water such that the concentration of calcium chloride is 25 mM was made the second partial reagent and the fourth partial reagent.

We have obtained the 9 kinds of the plasma of LA moderate positive patients, the 9 kinds of the plasma of LA weak positive patients, and 5 kinds of the plasma of the patients to whom warfarin has been given. As for the respective plasma, the one in which the normal plasma was mixed into the plasma of the patients at 1:1 was made measurement samples.

After two sets of 50 $\mu$L of the normal plasma and the respective measurement sample prepared as described above were prepared and 50 $\mu$L of the first partial reagent 1 (or the third partial reagent) was added to the respective sample of one set and heated to 37 degrees Centigrade for 3 minutes, 50 $\mu$L of the second partial reagent (or the fourth partial reagent) was added to the respective samples. The coagulation time was measured using the automatic blood coagulation analyzing apparatus Coagrex 800 (made by Shimadzu Corporation). Moreover, the coagulation time was measured similarly except that the first partial reagents 2-6 instead of the first partial reagent 1 were used.

The Lupus Ratio (LR value) indicated by the expression 1 described below was calculated from the coagulation time measured on the respective samples. The calculation of LR value was performed based on the literature (R. Schjetlein, et al., Thromb. Res. 1993, 69: 239-250).

[Expression 1]

Lupus Ratio (LR) = (b / a) / (d / c) = bc / ad

Here, a, b, c, and d in the expression 1 represent the measurement values obtained by the combination indicated in Table 1.

[Table1]

|  | Coagulation time during which the first partial reagent and the second partial reagent were used | Coagulation time during which the third partial reagent and fourth partial reagent were used |
|---|---|---|
| Patient plasma and normal plasma mixed at 1:1 | a | b |
| Normal plasma | c | d |

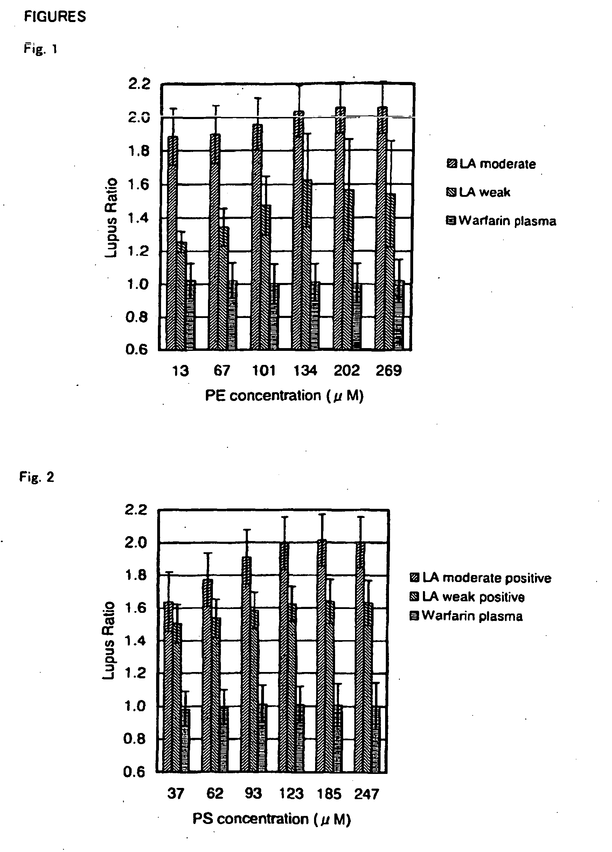

The results of the measurements are shown in Fig.1. Fig. 1 is a bar graph showing the average of LR values at the time when 9 kinds of the plasma of LA moderate positive patients, the plasma of LA weak positive patients and 5 kinds of the plasma of the patients to whom warfarin has been given were measured, and showing the standard deviation (SD) of LR values at the time when the respective specimens were measured by the T letter-shaped bar. Moreover, the abscissa shows the concentration of PE contained in the first partial reagents 1-6. As showing in Fig.1, it was clarified that the plasma of the patients to whom warfarin has been given and the plasma of LA weak positive patients and the plasma of LA moderate positive patients is capable of being distinguished.

(Example 2)

Consideration of the concentration of PS

The first partial reagents 7-12 and the third partial reagent were prepared by mixing HEPES, ellagic acid, Tris, phosphatidylserine (PS), phosphatidylethanolamine (PE) and phosphatidylcholine (PC).

Referring to the concentration of the respective components of the first partial reagent 7, the concentration of HEPES was 50 mM, the concentration of ellagic acid was 0.1 mM, the concentration of Tris was 25 mM, the concentration of PE was 67 $\mu$M, the concentration of PC was 38 $\mu$M, and the concentration of PS is 37 $\mu$M. Moreover, the first partial reagents 8-12 were prepared by carrying out the process similarly to the first partial reagent 7 except that the concentration of PS was changed into 62 $\mu$M, 93 $\mu$M, 123 $\mu$M, 185 $\mu$M, and 247 $\mu$M. The pH of the first partial reagents 7-12 was 7.35.

Referring to the concentration of the respective components of the third partial reagent, the concentration of HEPES was 50 mM, the concentration of ellagic acid was 0.1 mM, the concentration of Tris was 25 mM, the concentration of PS was 12 $\mu$M, the concentration of PE was 13 $\mu$M. and the concentration of PC was 38 $\mu$M. The pH was 7.35. It should be noted that a synthetic phospholipid whose aliphatic acid side chain is oleic acid was used for PE, PS and PC.

Moreover, the reagents prepared by purified water such that the concentration of calcium chloride is 25 mM was made the second partial reagent and the fourth partial reagent. The coagulation time was measured similarly to Example 1 except that the respective reagents prepared here are used.

The results of the measurements are shown in Fig.2. Fig. 2 is a bar graph showing the average of LR values at the time when 9 kinds of the plasma of LA moderate positive patients, 9 kinds of the plasma of LA weak positive patients and 5 kinds of the plasma of the patients to whom warfarin has been given were measured, and showing the standard deviation (SD) of LR values at the time when the respective specimens were measured by the T letter-shaped bar. Moreover, the abscissa shows the concentration of PE contained in the first partial reagents 7-12. As showing in Fig.2, it has been clarified that the plasma of the patients to whom warfarin has been given, and the plasma of LA weak positive patients and the plasma of LA moderate positive patients are capable of being distinguished. Moreover, the difference of LR value between the plasma of the patients to whom warfarin has been given and the plasma of the LA weak positive patients and the plasma of LA moderate positive patients became large until the concentration of PS contained in the first partial reagent reached up to 123 μM, but at the concentration of 123 μM or more, the difference of LR values became approximately certain.

(Example 3)

Consideration of reactivity of various kinds of specimens

The LA moderate positive specimens (specimen No.1-9 shown in Table 2), the LA weak positive specimens (specimen No. 10-17 shown in Table 2), the specimens of the patients to whom warfarin has been given (specimen No. 18-22 shown in Table 2), the specimens of the plasma of factor VIII deficiency patients (specimen No.23-25 shown in Table 2) and the specimens of the patients to whom heparin has been given (specimen No.26, 27 shown in Table 2) were measured using the reagents used in Example 1 (the first partial reagents 1-6, the second partial measurement reagent, the third partial measurement reagent, and the fourth partial measurement reagent) and the diluted Russell's viper venom reagent which is LA measurement reagent available in the market (dRVVT shown in Table 2).
The operation was carried out similarly to that of Example 1, and the LR value was measured. Moreover, the respective specimens using the diluted Russell's viper venom reagent were carried out by measuring the coagulation time according to the dosage regimen and calculating "ratio of the coagulation time". Moreover, the specimen in which "LR value", and "the ratio of the coagulation times" are 1.2 or more was determined as LA positive.
The results are indicated in Table 2.

[Table 2]

| | | dRVVT | First Partial reagent 1 | First partial reagent 2 | First partial reagent 3 | First partial reagent 4 | First partial reagent: 5 | First partial reagent 6 |
|---|---|---|---|---|---|---|---|---|
| 1 | LA moderate positive specimen 1 | 2.34 | 1.69 | 1.72 | 1.85 | 2.07 | 2.09 | 2.13 |
| 2 | LA moderate positive specimen 2 | 2.04 | 1.90 | 1.92 | 1.97 | 2.03 | 2.09 | 2.08 |
| 3 | LA moderate positive specimen 3 | 2.18 | 1.98 | 2.01 | 2.03 | 2.03 | 2.09 | 2.11 |
| 4 | LA moderate positive specimen 4 | 2.37 | 2.03 | 2.09 | 2.15 | 2.24 | 2.2.1 | 2.23 |
| 5 | LA moderate positive specimen 5 | 2.56 | 2.09 | 2.08 | 2.14 | 2.17 | 2.21 | 2.20 |
| 6 | LA moderate positive specimen 6 | 1.73 | 1.69 | 1.69 | 1.70 . | 1.69 | 1.70 . | 1.71 |

(continued)

| | | | dRVVT | First Partial reagent 1 | First partial reagent 2 | First partial reagent 3 | First partial reagent 4 | First partial reagent: 5 | First partial reagent 6 |
|---|---|---|---|---|---|---|---|---|---|
| 7 | LA moderate positive specimen 7 | | 2.71 | 2.08 | 2.09 | 2.09 | 2.08 | 2.11 | 2.11 |
| 8 | LA moderate positive specimen 8 | | 1.90 | 1.83 | 1.76 | 1.85 | 2.08 | 2.00 | 1.99 |
| 9 | LA moderate positive specimen 9 | | 1.46 | 1.68 | 1.72 | 1.83 | 1.95 | 2.01 | 1.97 |
| 10 | LA weak positive specimen 1 | | 1.31 | 1.34 | 1.42 | 1.45 | 1.57 | 1.48 | 1.40 |
| 11 | LA weak positive specimen 2 | | 1.68 | 1.33 | 1.56 | 1.79 | 2.11 | 2.11 | 2.10 |
| 12 | LA weak positive specimen 3 | | 1.29 | 1.21 | 1.26 | 1.26 | 1.36 | 1.32 | 1.36 |
| 13 | LA weak positive specimen 4 | | 1.54 | 1.20 | 1.24 | 1.40 | 1.43 | 1.39 | 1.31 |
| 14 | LA weak positive specimen 5 | | 1.46 | 1.26 | 1.31 | 1.34 | 1.46 | 1.36 | 1.35 |
| 15 | LA weak positive specimen 6 | | 1.17 | 1.16 | 1.23 | 1.67 | 2.01 | 1.98 | 2.00 |
| 16 | LA weak positive specimen 7 | | 1.12 | 1.28 | 1.35 | 1.45 | 1.57 | 1.52 | 1.45 |
| 17 | LA weak positive specimen 8 | | 1.33 | 1.27 | 1.38 | 1.42 | 1.49 | 1.37 | 1.36 |
| 18 | Warfarin patient specimen 1 | | 1.03 | 1.09 | 1.08 | 1.04 | 1.05 | 1.06 | 1.10 |

(continued)

| | | | dRVVT | First Partial reagent 1 | First partial reagent 2 | First partial reagent 3 | First partial reagent 4 | First partial reagent: 5 | First partial reagent 6 |
|---|---|---|---|---|---|---|---|---|---|
| | 19 | Warfarin patient specimen 2 | 0.94 | 1.03 | 1.03 | 1.03 | 1.05 | 1.05 | 1.09 |
| | 20 | Warfarin patient specimen 3 | 1.26 | 1.12 | 1.13 | 1.14 | 1.14 | 1.12 | 1.12 |
| | 21 | Warfarin patient specimen 4 | 1.34 | 1.09 | 1.09 | 1.11 | 1.12 | 1.11 | 1.17 |
| | 22 | Warfarin patient specimen 5 | 0.99 | 0.85 | 0.84 | 0.83 | 0.83 | 0.84 | 0.85 |
| | 23 | Factor VIII deficiency patient specimen 1 | 1.12 | 1.14 | 1.13 | 1.08 | 1.07 | 1.09 | 1.08 |
| | 24 | Factor VIII deficiency patient specimen 2 | 1.08 | 0.91 | 0.90 | 0.83 | 0.88 | 0.83 | 0.85 |
| | 25 | Factor VIII deficiency patient specimen 3 | 0.86 | 0.96 | 0.95 | 0.94 | 0.95 | 0.92 | 0.91 |
| | 26 | Heparin patient specimen 1 | 1.16 | 1.02 | 1.03 | 1.04 | 1.04 | 1.04 | 1.04 |
| | 27 | Heparin patient specimen 2 | 1.29 | 0.87 | 0.87 | 0.88 | 0.88 | 0.88 | 0.89 |

As indicated in Table 2, in the case where the diluted Russell's viper venom reagent was used, the specimen No. 15, 16 which are LA weak positive specimens were determined as negative, and the specimen No.20, 21 which are the specimens of the patients to whom warfarin has been given and the specimen No.27 which is the specimen of the patient to whom heparin has been given were determined as positive.

Moreover, in the case where the first partial reagent 1 whose concentration of PE is 13 $\mu$M was used, the specimen No. 15 which is the specimen of LA weak positive was determined as negative.

However, by using the first partial measurement reagents 2-6, it has been clarified that both of the specimen of LA moderate positive patients and the specimens of LA weak positive patients were determined as positive, the specimens of the patients to whom warfarin has been given, the specimens of the plasma of VIII factor deficiency patients and the specimens of the patients to whom heparin has been given were determined as negative as well as the specimens of the LA positive patients are distinguished from the plasma of the patients to whom heparin has been given and the specimen of the patients to whom warfarin has been given, and the detection sensitivity of LA has been enhanced and a reagent kit for detecting LA which can specifically detect LA even if it is a LA weak positive specimen can be provided.

**Claims**

1. A reagent kit for detecting lupus anticoagulant comprising:

   a first coagulation time measurement reagent containing phospholipid including phosphatidylserine and phosphatidylethanolamine; and
   a second coagulation time measurement reagent containing phospholipid including phosphatidylserine and phosphatidylethanolamine;

   wherein the concentration of phosphatidylethanolamine of said first coagulation time measurement reagent is in the range from 40 to 280 $\mu$M, and the concentration of phosphatidylethanolamine contained in said second coagulation time measurement reagent is in the range from 1 to 30 $\mu$M.

2. The reagent kit for detecting lupus anticoagulant according to Claim 1, wherein a concentration of phosphatidylethanolamine or phosphatidylserine in said first coagulation time measurement reagent is 3 to 15 times as high as a concentration of phosphatidylethanolamine or phosphatidylserine in said second coagulation time measurement reagent.

3. The reagent kit for detecting lupus anticoagulant according to Claim 1 or 2, wherein said first coagulation time measurement reagent and said second coagulation time measurement reagent contain phosphatidylcholine.

4. The reagent kit for detecting lupus anticoagulant according to any one of Claims 1 to 3, wherein said first coagulation time measurement reagent and said second coagulation time measurement reagent contain an activator and calcium ion.

5. The reagent kit for detecting lupus anticoagulant according to Claim 4, wherein said activator is selected from the group consisting of ellagic acid, kaolin, celite and colloidal silica.

6. The reagent kit for detecting lupus anticoagulant according to any one of Claims 1 to 3, wherein said first coagulation time measurement reagent and said second coagulation time measurement reagent contain snake venom and calcium ion.

7. The reagent kit for detecting lupus anticoagulant according to any one of Claims 1 to 3, wherein said first coagulation time measurement reagent and said second coagulation time measurement reagent contain a tissue factor and calcium ion.

8. A reagent kit for detecting lupus anticoagulant comprising:

   a first coagulation time measurement reagent comprising a first partial reagent and a second partial reagent; and
   a second coagulation time measurement reagent comprising a third partial reagent and a fourth partial reagent,

   wherein said first partial reagent contains phospholipid including phosphatidylserine and phosphatidylethanolamine, said second partial reagent contains a calcium ion, said third partial reagent contains phospholipid including phosphatidylserine and phosphatidylethanolamine, and said fourth partial reagent contain a calcium ion, the concentration of phosphatidylethanolamine contained in said first partial reagent is in the range from 40 to 280 $\mu$M, and the concentration of phosphatidylethanolamine contained in said third partial reagent is in the range from 1 to 30 $\mu$M.

9. The reagent kit for detecting lupus anticoagulant according to Claim 8, wherein a concentration of phosphatidylethanolamine or phosphatidylserine in said first partial reagent is 3 to 15 times as high as a concentration of phosphatidylethanolamine or phosphatidylserine in said third partial reagent.

10. The reagent kit for detecting lupus anticoagulant according to Claim 8 or 9, wherein said first partial reagent and said third partial reagent contain phosphatidylcholine.

11. The reagent kit for detecting lupus anticoagulant according to any one of Claims 8 to 10, wherein said first partial reagent and said third partial reagent contain an activator.

12. The reagent kit for detecting lupus anticoagulant according to Claim 11, wherein said activator is selected from the group consisting of ellagic acid, kaolin, celite and colloidal silica.

13. The reagent kit for detecting lupus anticoagulant according to any one of Claims 8 to 10, wherein said first partial reagent and said third partial reagent contain snake venom.

14. The reagent kit for detecting lupus anticoagulant according to any one of Claims 8 to 10, wherein said first partial reagent and said third partial reagent contain a tissue factor.

15. The reagent kit for detecting lupus anticoagulant according to any one of Claims 1 to 14, wherein said phospholipid is a synthetic phospholipid or at least 99% purified phospholipid derived from natural resource.

**Patentansprüche**

1. Reagenskit zum Detektieren von Lupus-Antikoagulans, umfassend:

   ein erstes Koagulationszeit-Meßreagens enthaltend Phospholipid umfassend Phosphatidylserin und Phosphatidylethanolamin; und
   ein zweites Koagulationszeit-Meßreagens enthaltend Phospholipid umfassend Phosphatidylserin und Phosphatidylethanolamin;

   worin die Konzentration von Phosphatidylethanolamin von besagtem ersten Koagulationszeit-Meßreagens im Bereich von 40 bis 280 $\mu$M ist, und die Konzentration von Phosphatidylethanolamin enthalten in besagtem zweiten Koagulationszeit-Meßreagens im Bereich von 1 bis 30 $\mu$M ist.

2. Reagenskit zum Detektieren von Lupus-Antikoagulans gemäß Anspruch 1, worin die Konzentration von Phosphatidylethanolamin oder Phosphatidylserin in besagtem ersten Koagulationszeit-Meßreagens 3 bis 15 mal so hoch ist wie die Konzentration von Phosphatidylethanolamin oder Phosphatidylserin in besagtem zweiten Koagulationszeit-Meßreagens.

3. Reagenskit zum Detektieren von Lupus-Antikoagulans gemäß Anspruch 1 oder 2, worin besagtes erstes Koagulationszeit-Meßreagens und besagtes zweites Koagulationszeit-Meßreagens Phosphatidylcholin enthalten.

4. Reagenskit zum Detektieren von Lupus-Antikoagulans gemäß einem der Ansprüche 1 bis 3, worin besagtes erstes Koagulationszeit-Meßreagens und besagtes zweites Koagulationszeit-Meßreagens einen Aktivator und Calciumionen enthalten.

5. Reagenskit zum Detektieren von Lupus-Antikoagulans gemäß Anspruch 4, worin besagter Aktivator ausgewählt ist aus der Gruppe bestehend aus Ellagsäure, Kaolin, Celit und kolloidalem Silica.

6. Reagenskit zum Detektieren von Lupus-Antikoagulans gemäß einem der Ansprüche 1 bis 3, worin besagtes erstes Koagulationszeit-Meßreagens und besagtes zweites Koagulationszeit-Meßreagens Schlangengift und Calciumionen enthalten.

7. Reagenskit zum Detektieren von Lupus-Antikoagulans gemäß einem der Ansprüche 1 bis 3, worin besagtes erstes Koagulationszeit-Meßreagens und besagtes zweites Koagulationszeit-Meßreagens einen Gewebefaktor und Calciumionen enthalten.

8. Reagenskit zum Detektieren von Lupus-Antikoagulans, enthaltend:

   ein erstes Koagulationszeit-Meßreagens umfassend ein erstes Teilreagens und ein zweites Teilreagens; und
   ein zweites Koagulationszeit-Meßreagens umfassend ein drittes Teilreagens und ein viertes Teilreagens,

   worin besagtes erstes Teilreagens Phospholipid umfassend Phosphatidylserin und Phosphatidylethanolamin enthält, besagtes zweites Teilreagens Calciumionen enthält, besagtes drittes Teilreagens Phospholipid umfassend Phosphatidylserin und Phosphatidylethanolamin enthält, und besagtes viertes Teilreagens Calciumionen enthält, wobei die Konzentration von Phosphatidylethanolamin enthalten in besagtem ersten Teilreagens im Bereich von 40 bis 280 $\mu$M liegt, und die Konzentration von Phosphatidylethanolamin enthalten in besagtem dritten Teilreagens im Bereich von 1 bis 30 $\mu$M liegt.

9. Reagenskit zum Detektieren von Lupus-Antikoagulans gemäß Anspruch 8, worin eine Konzentration von Phosphatidylethanolamin oder Phosphatidylserin in besagtem ersten Teilreagens 3 bis 15 mal so hoch ist wie die Konzentration von Phosphatidylethanolamin oder Phosphatidylserin in besagtem dritten Teilreagens.

10. Reagenskit zum Detektieren von Lupus-Antikoagulans gemäß Anspruch 8 oder 9, worin besagtes erstes Teilreagens und besagtes drittes Teilreagens Phosphatidylcholin enthalten.

11. Reagenskit zum Detektieren von Lupus-Antikoagulans gemäß einem der Ansprüche 8 bis 10, worin besagtes erstes Teilreagens und besagtes drittes Teilreagens einen Aktivator enthalten.

12. Reagenskit zum Detektieren von Lupus-Antikoagulans gemäß Anspruch 11, worin besagter Aktivator ausgewählt ist aus der Gruppe bestehend aus Ellagsäure, Kaolin, Celit und kolloidalem Silica.

13. Reagenskit zum Detektieren von Lupus-Antikoagulans gemäß einem der Ansprüche 8 bis 10, worin besagtes erstes Teilreagens und besagtes drittes Teilreagens Schlangengift enthalten.

14. Reagenskit zum Detektieren von Lupus-Antikoagulans gemäß einem der Ansprüche 8 bis 10, worin besagtes erstes Teilreagens und besagtes drittes Teilreagens einen Gewebefaktor enthalten.

15. Reagenskit zum Detektieren von Lupus-Antikoagulans gemäß einem der Ansprüche 1 bis 14, worin besagtes Phospholipid ein synthetisches Phospholipid ist oder ein zumindest 99 % aufgereinigtes Phospholipid abgeleitet aus natürlichen Quellen ist.

**Revendications**

1. Trousse de réactifs pour détecter un lupus anticoagulant comprenant :

   un premier réactif de mesure du temps de coagulation contenant des phospholipides y compris la phosphatidylsérine et la phosphatidyléthanolamine ; et
   un deuxième réactif de mesure du temps de coagulation contenant des phospholipides y compris la phosphatidylsérine et la phosphatidyléthanolamine ;

   dans laquelle la concentration de phosphatiyléthanolamine dudit premier réactif de mesure du temps de coagulation se situe dans la plage allant de 40 à 280 $\mu$M, et la concentration de phosphatidyléthanolamine contenue dans ledit deuxième réactif de mesure du temps de coagulation se situe dans la plage allant de 1 à 30 $\mu$M.

2. Trousse de réactifs pour détecter un lupus anticoagulant selon la revendication 1, dans laquelle une concentration de phosphatidyléthanolamine ou de phosphatidylsérine dans ledit premier réactif de mesure du temps de coagulation est de 3 à 15 fois aussi élevé qu'une concentration de phosphatidyléthanolamine ou de phosphatidylsérine dans ledit deuxième réactif de mesure du temps de coagulation.

3. Trousse de réactifs pour détecter un lupus anticoagulant selon la revendication 1 ou 2, dans laquelle le dit premier réactif de mesure du temps de coagulation et ledit deuxième réactif de mesure du temps de coagulation contiennent de la phosphatidylcholine.

4. Trousse de réactifs pour détecter un lupus anticoagulant selon l'une quelconque des revendications 1 à 3, dans laquelle ledit premier réactif de mesure du temps de coagulation et ledit deuxième réactif de mesure du temps de coagulation contiennent un activateur et l'ion calcium.

5. Trousse de réactifs pour détecter un lupus anticoagulant selon la revendication 4, dans laquelle ledit activateur est choisi dans le groupe constitué par l'acide ellagique, le kaolin, la célite et la silice colloïdale.

**6.** Trousse de réactifs pour détecter un lupus anticoagulant selon l'une quelconque des revendications 1 à 3, dans laquelle ledit premier réactif de mesure du temps de coagulation et ledit deuxième réactif de mesure du temps de coagulation contiennent du venin de serpent et l'ion calcium.

**7.** Trousse de réactifs pour détecter un lupus anticoagulant selon l'une quelconque des revendications 1 à 3, dans laquelle ledit premier réactif de mesure du temps de coagulation et ledit deuxième réactif de mesure du temps de coagulation contiennent un facteur tissulaire et l'ion calcium.

**8.** Trousse de réactifs pour détecter un lupus anticoagulant comprenant :

un premier réactif de mesure du temps de coagulation comprenant un premier réactif partiel et un deuxième réactif partiel ;
et
un deuxième réactif de mesure du temps de coagulation comprenant un troisième réactif partiel et un quatrième réactif partiel, dans laquelle ledit premier réactif partiel contient des phospholipides y compris la phosphatidylsérine et la phosphatidyléthanolamine, ledit deuxième réactif partiel contient l'ion calcium, ledit troisième réactif partiel contient des phospholipides y compris la phosphatidylsérine et la phosphatidyléthanolamine, et ledit quatrième réactif partiel contient l'ion calcium, la concentration de phosphatidyléthanolamine contenue dans ledit premier réactif partiel se situe dans la plage allant de 40 à 280 $\mu$M, et la concentration de phosphatidyléthanolamine contenue dans ledit troisième réactif partiel se situe dans la plage allant de 1 à 30 $\mu$M.

**9.** Trousse de réactifs pour détecter un lupus anticoagulant selon la revendication 8, dans laquelle une concentration de phosphatidyléthanolamine ou de phosphatidylsérine dans ledit premier réactif partiel est de 3 à 15 fois aussi élevée qu'une concentration de phosphatidyléthanolamine ou de phosphatidylsérine dans ledit troisième réactif partiel.

**10.** Trousse de réactifs pour détecter un lupus anticoagulant selon la revendication 8 ou 9, dans laquelle ledit premier réactif partiel et ledit troisième réactif partiel contiennent de la phosphatidylcholine.

**11.** Trousse de réactifs pour détecter un lupus anticoagulant selon l'une quelconque des revendications 8 à 10, dans laquelle ledit premier réactif partiel et ledit troisième réactif partiel contiennent un activateur.

**12.** Trousse de réactifs pour détecter un lupus anticoagulant selon la revendication 11, dans laquelle ledit activateur est choisi dans le groupe constitué par l'acide ellagique, le kaolin, la célite et la silice colloïdale.

**13.** Trousse de réactifs pour détecter un lupus anticoagulant selon l'une quelconque des revendications 8 à 10, dans laquelle ledit premier réactif partiel et ledit troisième réactif partiel contiennent du venin de serpent.

**14.** Trousse de réactifs pour détecter un lupus anticoagulant selon l'une quelconque des revendications 8 à 10, dans laquelle ledit premier réactif partiel et ledit troisième réactif partiel contiennent un facteur tissulaire.

**15.** Trousse de réactifs pour détecter un lupus anticoagulant selon l'une quelconque des revendications 1 à 14, dans laquelle ledit phospholipide est un phospholipide synthétique ou un phospholipide dérivé de source naturelle et purifié à au moins 99 %.

## FIGURES

### Fig. 1

Fig. 1

### Fig. 2

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1385003 A

**Non-patent literature cited in the description**

- **V. CHANTARANGKUL et al.** *Thromb. Res.,* 1992, vol. 67, 355-365
- **E. ROSNER et al.** *Thromb. Haemost.,* 1987, vol. 57, 144-147
- **E. ROSNER et al.** *Thromb. Haemast.,* 1987, vol. 57, 144-147
- **R. SCHJETLEIN et al.** *Thromb. Res.,* 1993, vol. 69, 239-250